# EUROPEAN PATENT SPECIFICATION

(11) **EP 1 681 023 B1**
(45) Date of publication and mention of the grant of the patent: **03.12.2008**
(21) Application number: 03778353.7
(22) Date of filing: 25.11.2003
(51) Int. Cl.: A61B 17/76

(54) **FIXING DEVICE FOR HIP OSTEOTOMIES AND FRACTURES IN CHILDREN AND ADOLESCENTS**
FIXIERVORRICHTUNG FÜR HÜFTOSTEOTOMIEN UND FRAKTUREN BEI KINDERN UND JUGENDLICHEN
DISPOSITIF DE FIXATION POUR DES OSTEOTOMIES DU BASSIN ET DES FRACTURES CHEZ DES ENFANTS ET DES ADOLESCENTS

(30) Priority: 23.09.2003 ES 200302202
(43) Date of publication of application: 19.07.2006
(73) Proprietor: Instituto Tecnologico de Canarias, S.A., 38009 Santa Cruz de Tenerife (ES); Recarte Garcia-Andrade, Enrique, 38009 Santa Cruz de Tenerife (ES); Monopoli Forleo, Donato, 38009 Santa Cruz de Tenerife (ES)
(72) Inventor: Recarte Garcia-Andrade, Enrique, 38009 Santa Cruz de Tenerife (ES); Monopoli Forlea, Donato, 38009 Santa Cruz de Tenerife (ES)
(74) Representative: Gomez-Acebo, Ignacio
(86) International application number: PCT/ES2003/000599
(87) International publication number: WO 2005/027764

(56) References cited:
- DE-U1- 8 213 228
- DE-U1- 9 000 161
- US-A- 3 489 143
- US-A- 5 484 439
- US-A- 5 749 872

## Description

### OBJECT OF THE INVENTION

The present invention relates to a device that has been specially designed as an implant to achieve simple and safe fixation of bone fragmente in osteotomies or fractures in children.

The device of the invention is applicable in the sphere of orthopaedic and traumatology surgery and introduces advantages in fixing the two fragments of bone created during a femur osteotomy in children or adolescents.

### BACKGROUND OF THE INVENTION

Traditionally, to fix the two fragments of femur following an osteotomy specific plates have been used whose operating principle is fundamentally based on the use of a cancellous screw placed in the axial direction to the femoral neck entering laterally under the greater trochanter, a screw which is fixed to a plate screwed laterally onto the femur.

The use of these plates entails problems that basically centre on the following aspects:
- On the one hand, it is difficult to achieve the correct pressure on the two bone fragments of the osteotomy's cuttig plane.
- On the other hand, it is also very difficult to achieve good establility during rotations of the bone's proximal fragment around the axis of the femoral neck.

Actually are known in the backgrounds of this invention several documents with relatively importance. The document US5749872 relates to a bone fracture fixator having a barrel configured for receiving both keyed and keyless lag screws in the same place. The document US5484439A relates to a modular implant for use in stabilizing femoral bone disorders resulting from injury disease or congenital defects. Both inventions use these plates for fixating the fractures, and mainly used in adults, because children do not suffer fractures which need plates. In opposite of this, the present invention relates to a fixation device for hip and fracture osteotomies, and therefore, only for children and teenagers.

The osteotomy is a control and clean fracture, with only two fragments, in order to modify, at least 30%, the angle of the femur's head (in general, less deformations do not need operation). In case of big deformation the femur's head does not develop and it can result in a necrosis, limp and others.

On the other hand, the fracture fixation plates, like those of documents US5749872A and US5484439A, are designed so as to use in unpredictable fractures, with a lot of fragments. Therefore, the design must have a plurality of holes for the fixation of all bone's fragments, and all of these holes are for fixation (see document US5484439A, holes of figure 5, 32a, 32b, 34).

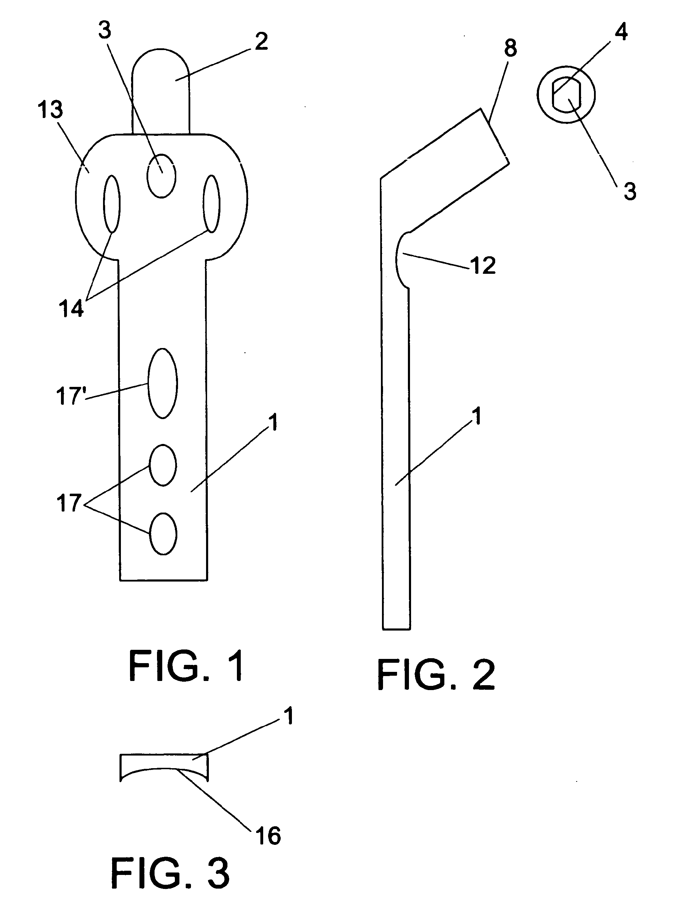

The plate of the document US5484439A is for modular fixation fractures, having a plurality of heads in several angles, and connecting with other plates bellow. However, the plate of the present invention is used for an osteotomy fixation correcting a surgical fracture so as to change the angle of the femur's head, which is formed wrong. Therefore, the holes of the plate (figure 1, hole 14) are not fixation screws, but anti-rotation.

### DESCRIPTION OF THE INVENTION

The fixation device proposed by the invention resolves the problems set out above in a fully satisfactory manner on the basis that the plate wherein this device fundamentally materializes presents proximal fixation on a minimun of two axes, which ensures stability during rotations while at the same time the plate provides a good travel course of its distal compression holes, ensuring the stability of the realtive positions of the fragments and good compression on the cutting plane.

For this purpose and more specifically, the plate wherein the device materializes presents as one of its characteristics the fact that in its proximal zone, in other words in the zone near the tubular ending of atatachaments to the femur's neck it incorporates a pair of lateral extensions designed with their respective orifices, which enable a staple or similar to be set through them, for fixation to the bone, which acts as an anti-rotation element for the porthesis.

In accordance with another of the invention's characteristics in this same area of the plate, in other words in the zone where the plate itself joins the tubular ending, said plate incorporates a notch or identation on its internal face which facilitates bending to adjust the angle between the plate and tubular ending to the specific requeriments of each case, with a view to ensuring that the device is of a universal nature.

At the same time, it is envisaged that the screw to be fixed to the femoral neck, in addition to the classical sector that determinates a telescopic screw which is the one that is geniuosly fixed to the bone, incorporates as an axial prolongation thereof an atacable arm with an anti-rotational nature on the plate's tubular ending, fitted with a stop for restricting penetration, in the shape of a cylindrical crown, located next to the screw, and equipped with an axial blind orifice, open towards its free end, whereby it receives, from the esternal or opposite side of the plate's tubular ending, a compression screw, with a suitable oversized head so as to rest against the opening of said ending, tractioning the telescopic screw against the against the latter.

This achieves, as mentioned earlier, good compression on the bone's cutting plane, since the prosthesis acts in traction on both its fragments and at the same time optium stability of the prosthesis in the face to tendencies for the rotation of the femoral neck's imaginary axis due to the effect or the two fixation points at a sufficient distance from each other, defined by the staple that passes thougth the plate's lateral orofices.

### DESCRIPTION OF THE DRAWINGS

To complement the description that is being made and with the aim of comtributing to a better understanding of the invention's characteristics, following a preferred practical embodiment thereof, a set of drawings is included as an integral part of said description, which. By way of illustration but not delimitation, shows the following:
- Figure 1: Shows a front elevation view of the plate that participates in the fixation device for hip and fracture osteotomies in children and adolescents, which constitutes the object of the present invention.
- Figure 2: Shows a profile of the palte of the preceding figure.
- Figure 3: Shows a cose-up in cross-section of the same plate.
- Figure 4: Shows a front side elevation view of the telescopic screw that complements the plate of the preceding figure
- Figure 5: Shows an axial view of the screw of figure 4.
- Figure 6: Shows a side elevation view of the compression screw.
- Figure 7: Shows an axial view of the screw of the preceding figure, from the end corresponding to its head.
- Figure 8: Shows a front elevation view of the staple that in turn complements the plate of figure 1 to 3.
- Figure 9: Finally, shows another view of the preceding figure's staple, from the free end of its sides.

### PREFERRED EMBODIMENT OF THE INVENTION

In view of the described figures, we can see how the following components participate in the device porposed by the invention: a plate (1) considerably lengthened, which at one of its ends extends into a tubular ending (2) that is configured against the plate (1) at an obtuse angle, as can particulary be seen from the profile drawing of figure 2, with the orifice (3) of the ending (2) presenting any non-cylindrical configuration, for example with both faces flat and opposing each other (4) as shown in the drawings to prevent the gyratory movement of the telescopic screw (5, 6, 7) that must be fitted into it with the possibility of relative axial desplacement.

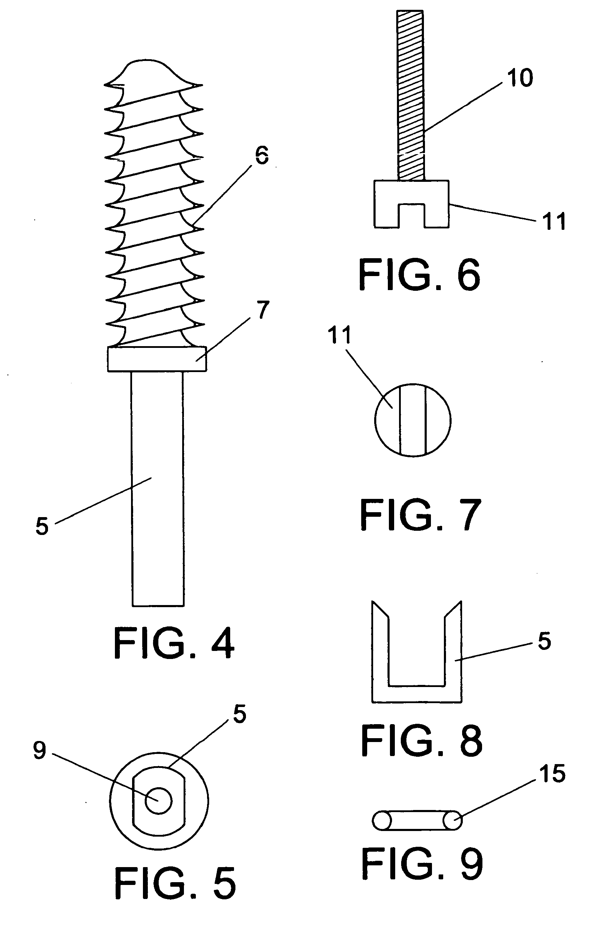

Said telescopic screw (5, 6, 7), shown in detail in figures 4 and 5 has a sector (6) that is configured as a cancellous screw for its insertion in the femora neck, finishing internally at one end in a stop (7) materialized in a cylindrical crown, for support on the free end (8) of the tubular ending (2) and establishing beyond said stop (7) an arm (5) with a section coinciding with the one of the orifice (3) of the tubular ending (2), as in turn can be seen from figure 5, with said arm (5) being provided with an axial, closed, threaded orifice (9) designed to accommodate a cmpression screw (10), finished with an oversize head (11) designed to rest on the external end of the orifice (3) of the tubula ending, corresponding to the zone where said ending is attached to the plate itself (1).

The plate (1), in said zone of attachment to the tubular ending (2) and on its internal face, incorporates a notch or indentation (12), especially visible in figure 2, which enables the plate to be bent in order to change the primitive angle between the plate (1) and the tubular ending (2) so that it can be adapted to the specific needs of each patient.

In this same zone, the plate (1) has two lateral extensions or wings (13) fitted with their respective orifices (14), which have been designed and positioned in order to allow the two side arms of a staple (15), such as the one shown in figures 8 and 9, or respective screws to pass throught them in order to fix small fragments.

To return once again to the plate (1), it has an internal face (16) with a slightly concave profile, as can be seen particulary in figure 3, for better adaptation to the femur, althougth noneless its profile is flat in correspondence with the indentation (12) to facilitate bending of the plate, with the plate (1) additionally incorporating in the distal zone various compression holes (17) for placing cortical screws on the distal fixation of the femur, one of which (17') has a greater travel course to ensure compression on the osteotomy's plane.

In accordance with this asembly, the method of operation of the device is as follows (said method not being part of the claimed invention):
- The telescopic screw (figure 4 and 5) is placed in the femoral neck leaving its arm (5) visible beneath the femur's cortical.
- Next, the osteotomy is made and the cervical-diaphisary angle is mesured.
- The plate (1) is bent along its notch or identation (12) to adapt it ti the previously measured angle and the tubular ending (2) is fitted to the arm (5) of the telescopic cancellous screw ensuring that the flat faces (4) of the internal surface of the orifice (3) of the tubular ending (2) and the external surface of the arm (5) of the screw coincide, previously called sector (6).
- The two faces of the cut are put into contact in the desired realative position and then distal screwing is proceeded with starting with the hole the longest travel course (17').
- The telescopic screw (figure 4 and 5) is fixed to the plate (1) with the help of a compression srew (10) whereby the two bone fragments become solidly joined.
- Finally, a staple (15) is inserted in the bone, throught the holes (14) of the plate (1), to immobilise the plate in the angular sense with regards to the femoral neck's axis.

## Claims

1. Fixation device for hip and fracture osteotomies in children and adolescents, comprising a plate and a telescopic screw (5,6,7), wherein the plate literally adapts to the femur, fixable through screwing thereof and finished at one of its ends in a tubular ending having one free end (8) and one orifice (3), said tubular ending forming an obtuse angle with the plate itself and which is designed to be fixed axially to the neck of the femur with the aid of said telescopic screw (5,6,7), wherein said plate (1) incorporates at its proximal end, the one corresponding to the location of the tubular ending (2), wings or side extensions (13) wherein respective orifices (14) are made acting as anti-rotational means for the plate, with the collaboration of means for fixation to the bone that pass through said orifices (14) **characterised in that** the telescopic screw (5,6,7) comprises a cancellous screw (6) finished at one of its ends in a stop (7) in the shape of a cylindrical crown, for support on said free end (8) and establishing beyond said stop (7) an arm (5) capable of telescopically playing inside said orifice (3).

2. Fixation device for hip and fracture osteotomies in children and adolescents, according to claim 1, **characterised in that** the plate itself (1) presents in its proximal end, specifically in the area of the tubular ending (2) and on its internal face a notch or indentation (12) that enables bending of the plate, specifically to modify the angle defined by the plate itself (1) and the tubular ending (2) of the plate.

## Patentansprüche

1. Befestigungsvorrichtung für Hüft- und Knochenbruch-Osteotomien an Kindern und Jugendlichen, welche eine Platte und eine teleskopische Schraube (5, 6, 7) umfasst, wobei die Platte sich im wahrsten Sinne des Wortes an das Femur anpasst, wobei diese durch das Anschrauben derselben fixierbar und an einem ihrer Enden in einem röhrenförmigen Ende ausgeführt ist, welches ein freies Ende (8) und eine Öffnung (3) besitzt, wobei das besagte röhrenförmige Ende einen stumpfen Winkel mit der Platte selbst formt und dazu ausgelegt ist, mittels der teleskopischen Schraube (5, 6, 7) axial mit dem Hals des Femurs befestigt zu werden, wobei die besagte Platte (1) an ihrem proximalen Ende, dem Ende, welches der Position des röhrenförmigen Endes (2) entspricht, Flügel oder seitliche Verlängerungen (13) einschließt, in denen entsprechende Öffnungen (14) ausgeführt sind, die als Antidrehmittel für die Platte dienen, mit der Hilfe von Mitteln zur Fixierung des Knochens, die durch die besagten Öffnungen (14) verlaufen, **dadurch gekennzeichnet, dass** die teleskopische Schraube (5, 6, 7) eine Spongiosaschraube (6) zur Abstützung auf dem besagten freien Ende (8) einschließt, und wobei jenseits des besagten Anschlags (7) ein Arm (5) festgelegt ist, der dazu in der Lage ist, teleskopisch innerhalb der besagten Öffnung (3) zu spielen.

2. Befestigungsvorrichtung für Hüft- und Knochenbruch-Osteotomien an Kindern und Jugendlichen nach Anspruch 1, **dadurch gekennzeichnet, dass** die Platte selbst (1) an deren proximalen Ende, im Besonderen im Bereich des röhrenförmigen Endes (2) und auf dessen innerer Seite, eine Einkerbung oder Vertiefung (12) aufweist, die eine Biegung der Platte ermöglicht, im Besonderen um den Winkel zu verändern, der von der Platte selbst (1) und dem röhrenförmigen Ende (2) der Platte definiert wird.

## Revendications

1. Dispositif de fixation pour hanche et ostéotomies de fracture chez les enfants et les adolescents, comprenant une plaque et une vis télescopique (5, 6, 7), dans lequel la plaque s'adapte totalement au fémur, à fixer par vissage de celle-ci et se terminant à l'une de ses extrémités par une terminaison tubulaire possédant une extrémité libre (8) et un orifice (3), ladite terminaison tubulaire formant un angle obtus avec la plaque elle-même et qui est conçu pour être fixé axialement au col du fémur à l'aide de ladite vis télescopique (5, 6, 7), dans lequel ladite plaque (1) comporte à son extrémité proximale, celle qui correspond à l'emplacement de la terminaison tubulaire (2), des ailettes ou extensions latérales (13) dans lesquelles des orifices respectifs (14) sont réalisés en guise de moyens empêchant la rotation de la plaque, en collaboration avec des moyens de fixation à l'os qui passent à travers lesdits orifices (14), **caractérisé en ce que** la vis télescopique (5, 6, 7) comprend une vis spongieuse (6) se terminant à l'une de ses extrémités en un arrêt (7) en forme de couronne cylindrique, pour un support sur ladite extrémité libre (8) et déterminant en dessous dudit arrêt (7) un bras (5) capable de jouer de manière télescopique à l'intérieur dudit orifice (3).

2. Dispositif de fixation pour hanche et ostéotomies de fracture chez les enfants et les adolescents, selon la revendication 1, **caractérisé en ce que** la plaque elle-même (1) présente à son extrémité proximale, spécifiquement dans la zone de la terminaison tubulaire (2) et sur sa face interne une encoche ou indentation (12) qui permet le pliage de la plaque, en particulier pour modifier l'angle défini par la plaque elle-même (1) et la terminaison tubulaire (2) de la plaque.
